# EUROPEAN PATENT APPLICATION

(11) **EP 4 501 378 A1**
(43) Date of publication of application: **05.02.2025**
(21) Application number: 23780904.1
(22) Date of filing: 30.03.2023
(51) Int. Cl.: A61M 5/31, A61J 1/06, A61M 5/00, A61M 5/28, A61M 5/315

(54) **SEALING BODY FOR SYRINGE**

(30) Priority: 31.03.2022 JP 2022060385
(71) Applicant: Kortuc Japan LLC, Tokyo 105-6004 (JP); Taisei Kako Co., Ltd., Kita-ku Osaka-shi Osaka 531-0072 (JP)
(72) Inventor: YAMASHITA, Shogo, Tokyo 105-6004 (JP); HORITA, Taiji, Ibaraki-shi, Osaka 567-0054 (JP); SONOYAMA, Tomoyuki, Ibaraki-shi, Osaka 567-0054 (JP); YOSHINAGA, Keisuke, Ibaraki-shi, Osaka 567-0054 (JP)
(74) Representative: Gulde & Partner
(86) International application number: PCT/JP2023/013242
(87) International publication number: WO 2023/190896

(57) **Abstract**

In syringes pre-filled with hydrogen peroxide solution for a long period, a problem of undesirable matter generation has become apparent. The purpose of the invention is to provide a syringe sealing, including a surface portion in contact with a hydrogen peroxide solution to be filled in the syringe, in which the syringe sealing is for suppressing generation of undesirable matter caused by hydrogen peroxide, and material of the surface portion is selected from the group consisting of fluoro rubber, fluororesin, silicone rubber and elastomer.

## Description

### Technical Field

The present invention relates to a syringe sealing, particularly a syringe sealing suitable for long-term storage of hydrogen peroxide solution in a syringe.

### Background

Hydrogen peroxide solutions are used as bleaching agents in industry and as disinfectants in the food industry. Hydrogen peroxide solution (trade name: Oxydol), which contains 2.5 to 3.5% (w/v) hydrogen peroxide, is used in medical applications as a disinfectant.

Hydrogen peroxide solution can be used as a radiosensitizer (Patent Document 1). Although hydrogen peroxide in hydrogen peroxide solution tends to decompose and generate gas, it has been found that hydrogen peroxide has low reactivity with cycloolefin polymers (COP) or cycloolefin copolymers (COC) (Patent Document 2).

### Prior arts

### Patent Documents

Patent Document 1: WO2008/041514
Patent Document 2: JP2020-81835A

### Summary of the Invention

### Problem to be Solved by the Invention

However, In syringes pre-filled with hydrogen peroxide solution for a long period, a problem of undesirable matter generation has become apparent. This issue has not been disclosed in either the Patent Document 1 or the Patent Document 2.

The inventors investigated materials that could cause the above undesirable matter and found that the above undesirable matter was caused by gaskets and caps manufactured from butyl rubber. After searching for materials that do not cause the above undesirable matter, the inventors discovered that by using gaskets and caps made of several materials in syringes filled with hydrogen peroxide solution, no undesirable matter is generated in the hydrogen peroxide solution, and then the invention was completed.

### Means for Solving the Problem

The purpose of the invention is to provide a syringe sealing, which has a surface portion in contact with a hydrogen peroxide solution to be filled in the syringe,
in which the syringe sealing is for suppressing generation of undesirable matter caused by hydrogen peroxide, and
material of the surface portion is selected from the group consisting of fluoro rubber, fluororesin, silicone rubber and elastomer.

The syringe sealing can be used to suppress the generation of undesirable matter in the syringe that have been pre-filled with the hydrogen peroxide solution for a long period.

The syringe sealing may include a gasket and a cap.

The another purpose of the invention is to provide a syringe equipped with the syringe sealing, in which the syringe is for suppressing the generation of undesirable matter caused by the hydrogen peroxide.

The syringe can be used to suppress the generation of undesirable matter in the syringe that have been pre-filled with the hydrogen peroxide solution for a long period.

The syringe above may be pre-filled with hydrogen peroxide solution.

### Brief Description of the Drawing

Fig. 1A schematically shows a pre-filled syringe filled with hydrogen peroxide solution, according to the present embodiment.
Fig. 1B shows an end view of a cap having a cap sealing member provided at the inside bottom of cap.
Fig. 1C shows an end view of a cap for another embodiment, having a cap body and a cap sealing member provided at the inside bottom of cap body.
Figs. 2A and 2B are photographs of gaskets manufactured with butyl rubber.
Figs. 3A and 3B show photographs of gaskets laminated with polytetrafluoroethylene (top surface laminated gaskets) on the top surface of each gasket (top surface in contact with the hydrogen peroxide solution).
Figs. 4A and 4B are photographs of gaskets manufactured with fluoro rubber.
Figs. 5A and 5B show photographs of gaskets manufactured with polytetrafluoroethylene for the surface in contact with the hydrogen peroxide solution.
Figs. 6A and 6B are photographs of the inside bottoms of caps manufactured with butyl rubber.
Figs. 7A and 7B are photographs of gaskets manufactured with silicone rubber.
Figs. 8A and 8B are photographs of gaskets manufactured with styrenic thermoplastic elastomer.
Figs. 9A and 9B are photographs of gaskets manufactured with styrenic thermoplastic elastomer.

### Description of Embodiments

### Definition

For convenience, certain terms employed in the context of the present disclosure are collected here. Unless defined otherwise, all technical and scientific terms used herein have the same meaning as commonly understood by one of the ordinary skilled in the art to which this invention belongs. The singular forms "a", "an", and "the" are used herein to include plural referents unless the context clearly dictates otherwise.

Notwithstanding that the numerical ranges and parameters setting forth the broad scope of the invention are approximations, the numerical values set forth in the specific examples are described as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art.

Hereinafter, embodiments of the present invention are illustrated in detail. The following embodiments are illustrative only and do not limit the scope of the present invention. In order to avoid redundancy, explanation for similar contents is not repeated.

### Pre-Filled Syringe 1

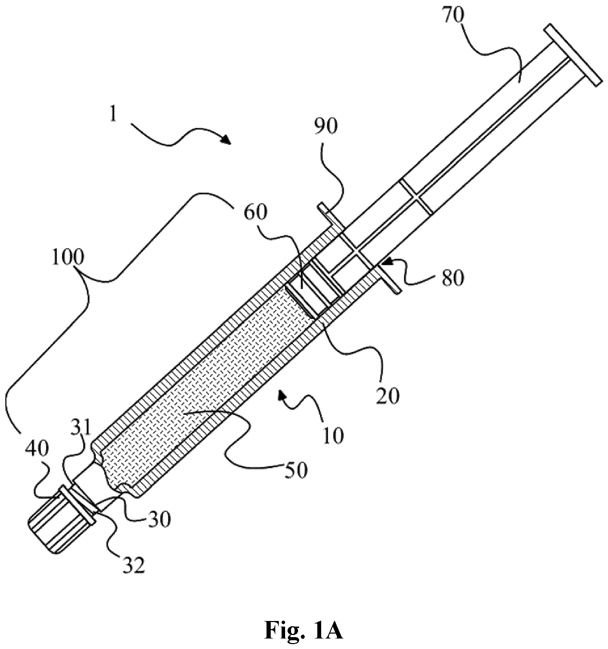

Fig. 1A schematically shows a syringe 10 filled with a hydrogen peroxide solution 50 (hereinafter referred to as "pre-filled syringe 1"). The syringe 10 has a barrel 20, a syringe sealing 100 (a cap 40 and a gasket 60), and a plunger rod 70. One end of the syringe 10 has a needle attachment portion 30 into which the hydrogen peroxide solution 50 is drained, and the other end of the syringe 10 has an opening 80 for inserting the plunger rod 70, and a flange 90 around the opening 80. The flange 90 may not be provided. The pre-filled syringe 1 shown in Fig. 1A has the cap 40 provided on the needle attachment portion 30 and the plunger rod 70 with the gasket 60 inserted into the opening 80 to seal the filled hydrogen peroxide solution 50.

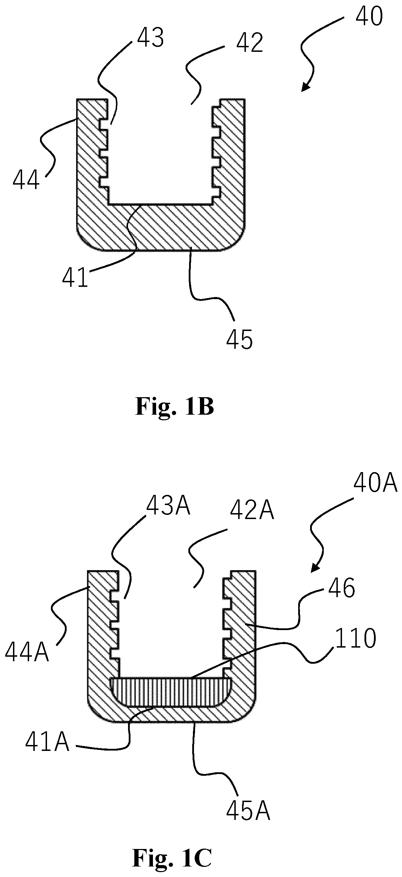

Fig. 1B shows an end view of the cap 40. Fig. 1B schematically shows a face of the cap 40 when the cap 40 is cut along the longitudinal central axis of the syringe 10 equipped with the cap 40. The cap 40 includes a cylindrical wall 44, an insertion opening 42 provided at one end of the cylindrical wall 44, and a sealing portion 45 provided at the other end of the cylindrical wall 44. A helical groove 43 is present on the inner wall of the cap 40 and is capable of screwing with a screw thread 32 of the outer wall 31 of the needle attachment portion 30. The helical groove 43 may not be present on the inner wall of the cap 40. If the helical groove 43 is not present on the inner wall of the cap 40, there is no screw thread 32 on the outer wall 31 of the needle attachment portion 30, and the needle attachment portion 30 can fit by friction within the cap 40. When the needle attachment portion 30 is inserted into the cap 40, the tip of the needle attachment portion 30 contacts the bottom 41 of the sealing portion 45 to suppress the discharge of the hydrogen peroxide solution 50 from the needle attachment portion 30.

Fig. 1C shows a cap 40A for another embodiment. Fi. 1C shows an end view of a cap body 46 and a cap sealing member 110 provided at the inside bottom 41A of the cap body 46. The cap 40A shown in Fig. 1C is common to the cap 40 shown in Fig. 1B, except for the cap sealing member 110. The cap body 46 includes a cylindrical wall 44A, an insertion opening 42A provided at one end of the cylindrical wall 44A, and a sealing portion 45A provided at the other end of the cylindrical wall 44A. A helical groove 43A is present on the inner wall of the cap body 46 and in capable of screwing with the screw thread 32 of the outer wall 31 of the needle attachment portion 30. The helical groove 43A may not be present on the inner wall of the cap body 46. If the helical groove 43A is not present on the inner wall of the cap body 46, there is no screw thread 32 on the outer wall 31 of the needle attachment portion 30, and the needle attachment portion 30 can fit by friction into the cap body 46. When the needle attachment portion 30 is inserted into the cap body 46, the tip of the needle attachment portion 30 contacts the cap sealing member 110 to suppress the discharge of the hydrogen peroxide solution 50 from the needle attachment portion 30.

In this embodiment, the hydrogen peroxide solution 50 refers to a solution of hydrogen peroxide dissolved in a solvent (e.g., water) and may contain additives (e.g., phosphoric acid and phenacetin) as needed. In this embodiment, the barrel 20 may be manufactured from a single material or may be composed of multiple materials (including multi-layered structures such as coatings). In the case of barrel 20 manufactured from a single material, the entire barrel 20 is made of a resin. In the case of a barrel 20 made from multiple materials, a portion of the barrel 20 in direct contact with the hydrogen peroxide solution 50 may be made of the resin, and the other portions may be made of a material with high hydrogen peroxide decomposition, such as glass. In this embodiment, cycloolefin polymer (COP), cyclo-olefin copolymer (COC), and polypropylene may be examples of the resin.

In this embodiment, the concentration of hydrogen peroxide in the hydrogen peroxide solution in the pre-filled syringe is, for example, 0.01, 0.02, 0.03, 0.04, 0.05, 0.06, 0.07, 0.08, 0.09, 0.1, 0.5, 1, 5, 10, 15, 20, 25, 30, 35 or 40 and may be in the range between any two values selected from the above group, e.g., from 0.01 to 40% (v/v), preferably from 0.05 to 30% (v/v).

### Syringe Sealing 100

The syringe sealing 100 suppresses the generation of undesirable matter caused by thehydrogen peroxide. The syringe sealing 100 includes a surface portion in contact with the hydrogen peroxide solution 50 to be filled in the syringe 10. The material of the surface portion is selected from the group consisting of fluoro rubber, fluororesin, silicone rubber, and elastomer. The material of the syringe sealing 100 may be selected from the group consisting of fluoro rubber, fluororesin, silicone rubber, and elastomer.

In another embodiment, the syringe 10 including the syringe sealing 100, the syringe 10 being for suppressing the generation of undesirable matter caused by the hydrogen peroxide is provided.

In another embodiment, the pre-filled syringe 1 filled with hydrogen peroxide solution 50 is provided.

The syringe sealing 100 may be composed of a surface portion and a body portion (i.e., it may be composed of two or more elements). The material of the surface portion is selected from the group consisting of fluoro rubber, fluororesin, silicone rubber, and elastomer. It is preferable that the body portion dose not come in contact with the hydrogen peroxide solution 50 to be filled in the syringe 10. The material of the body portion may be the same as the material of the surface portion. If the material of the body portion is the same as the material of the surface portion, the body portion may be integrally molded with the surface portion (i.e., the syringe sealing 100 may be composed of only one element). If the body portion does not come in contact with the hydrogen peroxide solution 50 to be filled in the syringe 10, the material of the body portion may be a material (e.g., butyl rubber) that generates undesirable matter when in contact with the hydrogen peroxide solution 50. The syringe sealing 100 may include the gasket 60 and the cap 40. In one embodiment, the syringe sealing 100 is the gasket 60. In another embodiment, the syringe sealing 100 is the cap 40. If the syringe sealing 100 is the cap 40, the cap 40 may include the surface portion and the body portion. If the cap 40 includes the surface portion and the body portion, the surface portion is the cap sealing member 110 and the body portion is the cap body 46.

### Undesirable Matter

The expression "undesirable matter caused by hydrogen peroxide" is solid matter caused by the hydrogen peroxide solution 50 filled in the syringe 10, which may be introduced into living body with the hydrogen peroxide solution 50 or may inhibit the discharge of the hydrogen peroxide solution 50 from within syringe 10 when the hydrogen peroxide solution 50 is administered to the living body. The "undesirable matter" is derived from a rubber (e.g., butyl rubber). In some embodiments, the syringe sealing 100 suppresses the generation of undesirable matter caused by contact with hydrogen peroxide for a long period (e.g., at least 1 month, at least 3 months, and at least 6 months). In some embodiments, the syringe sealing 100 suppresses the generation of undesirable matter caused by contact with hydrogen peroxide for a certain period (e.g., at least 6 months, at least 3 months, and at least 1 month). The expression "suppress the generation of undesirable matter" means "suppress the generation of undesirable matter to the extent that the existence of undesirable matter does not adversely affect the administration of the hydrogen peroxide solution to a living body" or "suppress the generation of undesirable matter to the extent that the undesirable matter is undetectable". The expression "to the extent that the undesirable matter is undetectable" is determined based on the capability of analytical instruments used in quality control in the art.

### Fluoro Rubber

Fluoro rubbers include, for example, a copolymer of vinylidene fluoride (VDF) and hexafluoropropylene (HFP) (binary FKM), a copolymer of vinylidene fluoride (VDF), hexafluoropropylene (HFP) and tetrafluoroethylene (TFE) (ternary FKM), a copolymer of tetrafluoroethylene (TFE) and propylene (Pr) (FEP), a copolymer of vinylidene fluoride (VDF), propylene (Pr) and tetrafluoroethylene (TFE), a copolymer of ethylene (E) and tetrafluoroethylene (TFE) (ETFE) E), a copolymer of tetrafluoroethylene (TFE) and perfluoromethyl vinyl ether (PMVE), a copolymer of vinylidene fluoride (VDF), tetrafluoroethylene (TFE) and perfluoromethyl vinyl ether (PMVE), vinylidene fluoride (VDF) and a copolymer of perfluoromethyl vinyl ether (PMVE), copolymer of tetrafluoroethylene (TFE) and perfluoroalkyl ether (PFAE). It is preferable to use one or more of these.

### Fluororesin

Fluororesin includes, for example, polytetrafluoroethylene (PTFE), ethylene tetrafluoroethylene (ETFE) copolymer, perfluoroalkoxyalkane (PFA), perfluoroethylene propane (FEP) copolymer, polyvinylidene fluoride (PVDF), polychlorotrifluoroethylene (PCTFE), ethylene chlorotrifluoroethylene (ECTFE) copolymer.

### Silicone Rubber

Silicone rubber is, for example, silicone rubber obtained by curing a silicone rubber composition (cured silicone rubber), and silicone rubber with a hardness of 20 to 80, preferably 50 to 70, on a Durometer A hardness tester as specified in JIS K 6249.

### Elastomer

Styrenic elastomers can be used as elastomers. The styrenic elastomers can include SBS (styrene-butadiene-styrene), SIS (styrene-isoprene-styrene), SEBS (styrene-ethylene-butadiene-styrene), SIS (styrene-isoprene-styrene), and SEPS (styrene-ethylene-propylene-styrene). The material of the surface portion may be an elastomer (e.g., styrenic elastomer). The elastomer may be a thermoplastic elastomer.

### Other Embodiments

A gasket for suppressing the generation of undesirable matter caused by hydrogen peroxide is provided, in which a material of the gasket is selected from the group consisting of fluoro rubber, fluororesin, silicone rubber, and elastomer. The gasket is attached to the tip of the plunger rod for the syringe.

There is provided a cap for suppressing the generation of undesirable matter caused by hydrogen peroxide, in which the cap has a surface portion in contact with the hydrogen peroxide solution to be filled in the syringe, and in which a material of the surface portion is selected from the group consisting of fluoro rubber, fluororesin, silicone rubber, and elastomer. The cap according to this embodiment is attached to the syringe.

### EXAMPLES

### Test 1

We investigated materials for suppressing the generation of undesirable matter caused by hydrogen peroxide. First, gaskets (n=10) were manufactured with the materials listed in Table 1.

**[Table 1]**

| | | |
|---|---|---|
| Experimental Example 1 | Chlorobutyl rubber | 2.25mL Pi 1 (Taisei Kako) |
| Experimental Example 2 | Laminated top surface | 2.25mL PLP (Taisei Kako) |
| Experimental Example 3 | Fluoro rubber | Fluoro Rubber Piston (Taisei Kako) |
| Experimental Example 4 | Polytetrafluoroethylene (PTFE) -silicone rubber | ClearX (Taisei Kako) |

Each gasket was fitted to a plunger rod, and eahc plunger rod with the gasket was attached to ta barrel of a syringe. A hydrogen peroxide solution was filled into each syringe and each syringe was sealed with a cap. The cap was manufactured from chlorobutyl rubber (Experimental Example 5). Each syringe was stored in a thermostatic chamber set at 60 °C and 75% RH (relative humidity) for 3 months. A syringe not filled with hydrogen peroxide solution was used as a control. After 3 months, each gasket was observed with a microscope.

### Results of Test 1

### Regarding Experimental Example 1

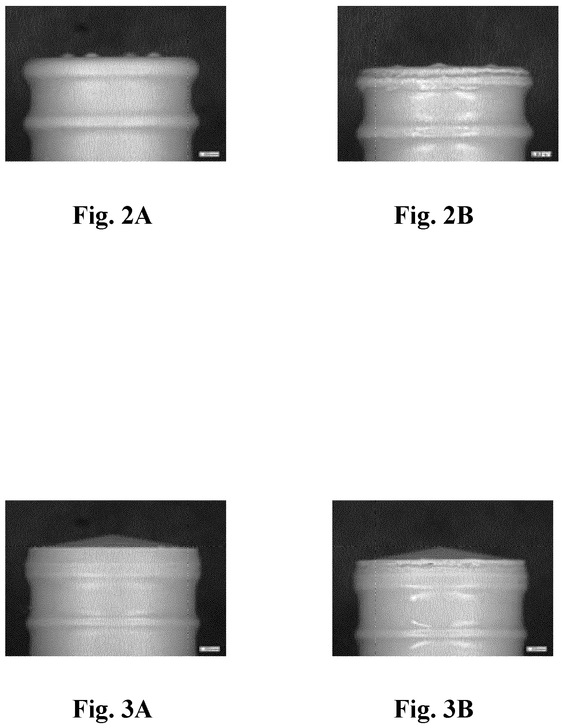

Figs. 2A and 2B are photographs of the gaskets manufactured with chlorobutyl rubber. Fig. 2A is the photograph of the control. The gasket manufactured with chlorobutyl rubber produced undesirable matter on the surface in contact with the hydrogen peroxide solution (Fig. 2B). The same result was observed for the remaining nine gaskets (not shown).

### Regarding Experimental Example 2

Figs. 3A and 3B are photographs of the gaskets manufactured with chlorobutyl rubber, each gasket having the top surface (top surface in contact with the hydrogen peroxide solution) laminated with polytetrafluoroethylene (PTEF) (top surface laminated gasket). Fig. 3Ais the photograph of the control. The top surface laminated gasket did not produce undesirable matter on the top surface (Fig. 3B) but did produce undesirable matter on the unlaminated surface (Fig. 2B). The same results were observed for the remaining nine gaskets (not shown).

### Regarding Experimental Example 3

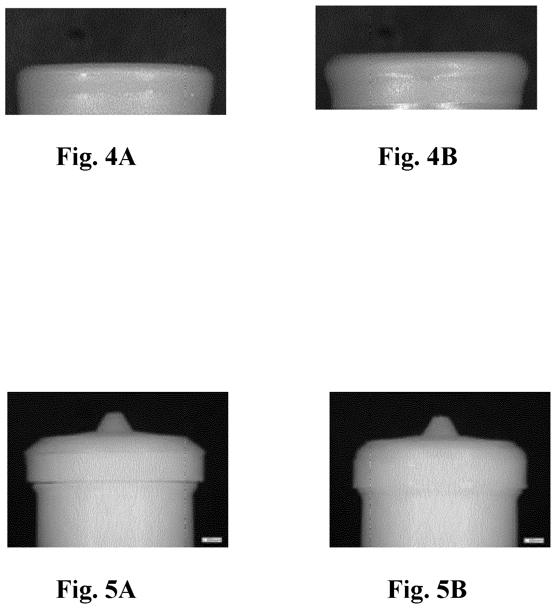

Figs. 4A and 4B are photographs of the gaskets manufactured with fluoro rubber. Fig. 4A is the photograph of the control. No undesirable matter is observed in the gasket manufactured with fluoro rubber (Fig. 4B). The same results were observed for the remaining nine gaskets (not shown).

### Regarding Experimental Example 4

Figs. 5A and 5B show the photographs of the gaskets (ClearX) having a body portion of which surface in contact with the hydrogen peroxide solution is made of polytetrafluoroethylene (PTFE) and a sealing member manufactured with silicone rubber containing ultra-high molecular weight polyethylene. Fig. 5Ais the photograph of the control. No undesirable matter was observed in the gaskets manufactured with polytetrafluoroethylene and silicone rubber (Fig. 5B). The same results were observed for the remaining nine gaskets (not shown).

### Regarding Experimental Example 5

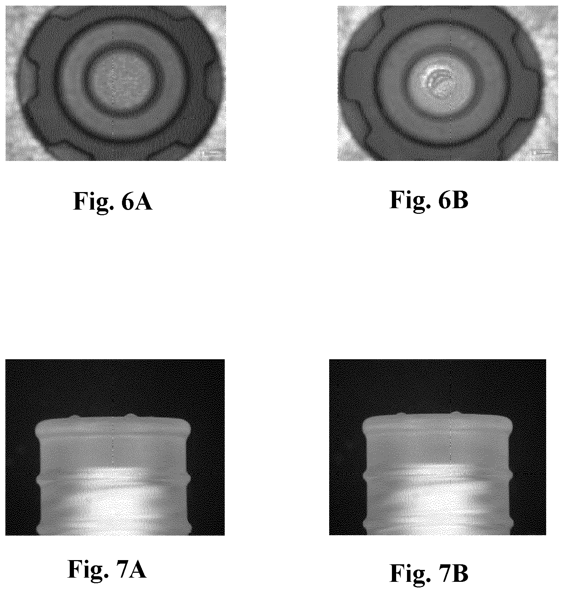

Figs. 6A and 6B are the photographs of the inside bottom of the caps manufactured with chlorobutyl rubber. Fig. 6A is the photograph of the control. Undesirable matter is produced on the inner bottom (i.e., the surface in contact with the hydrogen peroxide solution) of the caps manufactured with chlorobutyl rubber (Fig. 6B). The same result was observed for the remaining nine caps (not shown).

### Test 2

Next, gaskets were manufactured with the materials listed in Table 2 (n=10). The gaskets were stored under the same conditions as in Test 1, except that the storage period was set to 1 month. 1 month later, the gaskets were observed under a microscope.

**[Table 2]**

| | | |
|---|---|---|
| Experimental example 6 | Silicone rubber | 2.25mL Pi 2 (Si) (Taisei Kako) |

### Results of Test 2

### Regarding Experimental Example 6

Fig. 7A shows a photograph of silicone rubber before storage in the hydrogen peroxide solution, and Fig. 7B shows a photograph of silicone rubber after storage in the hydrogen peroxide solution. It was clear that no undesirable matter is generated on silicone rubber after storage in the hydrogen peroxide solution at 60°C for one month. The same results were obtained for the remaining nine silicone rubbers (not shown).

### Test 3

Next, gaskets were manufactured with the materials shown in Table 3 (Experimental example 7: n=10, Experimental example 8: n=1). They were stored under the same conditions as in Test 1. After each storage period, each material was observed with a microscope.

**[Table 3]**

| | | |
|---|---|---|
| Experimental Example 7 | Styrenic thermoplastic elastomer | PJ6300C (Mitsubishi Chemical Corporation) |
| Experimental Example 8 | Styrenic thermoplastic elastomer | PJ5302B (Mitsubishi Chemical Corporation) |

### Results of Test 3

### Regarding Experimental Example 7

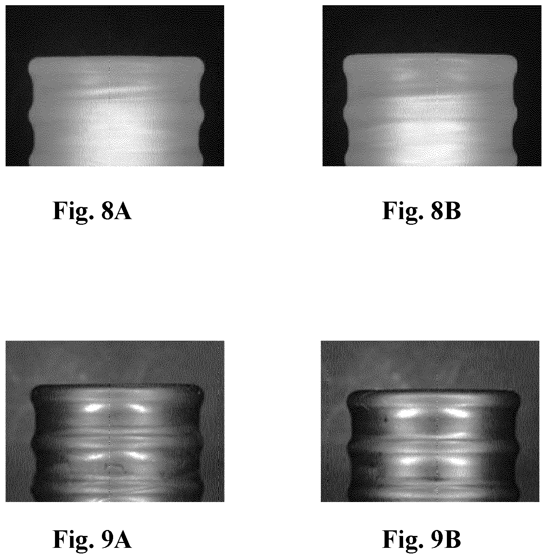

Fig. 8A shows a photograph of styrenic thermoplastic elastomer before storage in the hydrogen peroxide solution, and Fig. 8B shows a photograph of styrenic thermoplastic elastomer after storage in the hydrogen peroxide solution. It is clear that no undesirable matter is generated on the styrenic thermoplastic elastomers after storage in the hydrogen peroxide solution at 60° C for three months. The same results were obtained for the remaining nine styrenic thermoplastic elastomers (not shown).

### Regarding Experimental Example 8

Fig. 9A shows a photograph of styrenic thermoplastic elastomer before storage in the hydrogen peroxide solution, and Fig. 9B shows a photograph of styrenic thermoplastic elastomer after storage in the hydrogen peroxide solution. It is clear that no undesirable matter is generated on the styrenic thermoplastic elastomer after storage in the hydrogen peroxide solution at 60° C for one month.

### EXPLANATION OF REFERENCES

- 1: Pre-filled syringe
- 10: Syringe
- 20: Barrels
- 30: Needle attachment portion
- 31: Outer wall
- 32: Screw thread
- 40, 40A: Cap
- 41, 41A: Bottom
- 42, 42A: Insertion opening
- 43, 43A: Helical groove
- 44, 44A: Cylindrical wall
- 45, 45A: Sealing portion
- 45 46: Cap body
- 50: Hydrogen peroxide solution
- 60: Gasket
- 70: Plunger rod
- 80: opening
- 90: Flange
- 100: Syringe sealing
- 110: Cap sealing member

## Claims

1. A syringe sealing, comprising a surface portion in contact with a hydrogen peroxide solution to be filled in the syringe,
wherein the syringe sealing is for suppressing generation of undesirable matter caused by hydrogen peroxide, and
material of the surface portion is selected from the group consisting of fluoro rubber, fluororesin, silicone rubber and elastomer.

2. The syringe sealing according to claim 1, the syringe sealing comprises a gasket and a cap.

3. A syringe equipped with the syringe sealing according to claim 1 or 2, wherein the syringe is for suppressing the generation of undesirable matter caused by the hydrogen peroxide.

4. The syringe according to claim 3, the syringe is pre-filled with hydrogen peroxide solution.
